# EUROPEAN PATENT APPLICATION

(11) **EP 3 628 336 A1**
(43) Date of publication of application: **01.04.2020**
(21) Application number: 19181264.3
(22) Date of filing: 19.06.2019
(51) Int. Cl.: A61K 51/12, A61K 49/00, A61K 49/04, A61K 49/18, A61K 103/32, A61P 35/00

(54) **PHARMACEUTICAL COMPOSITION COMPRISING AN OXIDE OF YTTRIUM SUPPORTED ON A SULFUR-CONTAINING CARBON-BASED PARTICLE FOR USE IN THERAPEUTIC APPLICATIONS**

(30) Priority: 20.06.2018 EP 18178743
(71) Applicant: QUIREM Medical BV, 7418 AH Deventer (NL)
(72) Inventor: BRANDTS, Jim, 3454 PK De Meern (NL); BERBEN, Peter, 3454 PK De Meern (NL); SCHLESINGER, Maik, 67056 Ludwigshafen (DE); WAGEMAKER, Lorianne, 3454 PK De Meern (NL); NIJSEN, Johannes Franciscus Wilhelmus, 7418 AH Deventer (NL)
(74) Representative: Féaux de Lacroix, Stefan

(57) **Abstract**

The invention is directed to a pharmaceutical composition, comprising a body comprising an oxide of yttrium, wherein preferably the oxide of yttrium is in the form of (nano)particles, supported on a sulfur-containing carbon-based particle, wherein said body comprises yttrium to sulfur in an atomic ratio ranging from 1:0.01 to 1:10, and preferably a pharmaceutically acceptable carrier, adjuvant, diluent and/or excipient.

## Description

The invention is directed to a pharmaceutical composition comprising a body comprising oxides of yttrium (Y₂O₃), which are supported on a sulfur-containing carbon-based particle, and its use in therapeutic applications.

Yttrium can be used in selective radiation therapy for the treatment of primary and metastatic hepatic malignancies. Upon neutron irradiation ⁸⁹Y is converted to the radioactive isotope ⁹⁰Y, which is a beta-radiation emitter. Kennedy, A. et al. Int. J. Radiat. Oncol. Biol. Phys. 2007, 68, 13-23 has shown that sufficient evidence exists to support the safety and effectiveness of ⁹⁰Y microsphere therapy.

Yttrium is particularly attractive since it is a high energy beta emitter when irradiated to Yttrium-90 (⁹⁰Y) and has a half-life of 64.2 hours. Consequently, it can be used in radioablation. Further, it is known in the art that yttrium can be visualized by positron emission tomography (PET) due to the fact that a small portion of decay is through pair production (D'Arienzo M., Atoms 2013 1(1), 2-12). Additionally, Brehmstrahlung is produced during the decay which can also be used for imaging purposes (Simon N. et al., Radiology 1967, 88, 719-724).

Various studies have been conducted to locally administer radionuclides, such as radioactive isotopes of yttrium, particularly as a treatment for cancer with promising results (Dutton S.J. et al. BMC Cancer 2014; 14; 497)

WO-A-02/34300 describes a particulate material comprising a polymer matrix, in particular an ion exchange resin, and a radionuclide, a method for the preparation thereof and use of this particulate material. WO-A-02/34300 describes preparing this particulate material by adsorbing a radionuclide onto a polymer matrix and precipitating the radionuclide as an insoluble salt to stably incorporate the radionuclide into the polymer matrix. The manufacturer does not recommend exposing the microspheres to ionic solutions (ionic contrast, saline). The initial patent application for the resin microspheres indicated that 0.01% to 0.4% of ⁹⁰Y is released from the microsphere after 20 minutes in water (Westcott M.A. et al. Adv. Radiation Onco. 2016, 1, 351-364).

Chamberlain M N et al.: "Hepatic Metastases - A Physiological Approach to Treatment", British Journal of Surg, John Wright & Sons, Bristol, GB, vol. 70, no. 10, 1 October 1983 (1983-10-01), pages 596-598, discloses microspheres comprising radioactive yttrium for use in the treatment of hepatic metastases. The activated yttrium is incorporated into styrene-divinylbenzene copolymer ion exchange resin microspheres. There is no revelation of a sulfur content of the body.

WO 86/03124 describes microspheres of solid glass that contain the element yttrium-89. The microspheres can be activated in a neutron beam to ⁹⁰Y-micropspheres. The disadvantages of these spheres are the relatively high density and the presence of other radioactive components due to the presence of alumina and silica that are activated to unwanted radionuclides.

It is an object of the invention to provide particles comprising oxides of yttrium on a particulate support with improved properties over known materials, in particular (i) with respect to improving the stability of the yttrium oxides in a carrier material in a liquid, such as an aqueous solution or a biological fluid (e.g. blood), especially under neutral and acidic conditions and (ii) with respect to providing materials with lower density, which enables their use in therapeutic applications.

It was found that these objects can be realized by a body comprising an oxide of yttrium, wherein preferably the oxide of yttrium is in the form of nanoparticles, supported on a sulfur-containing carbon based particle, for use as a medicament and/or diagnostic agent.

The objects are furthermore realized by a pharmaceutical composition, comprising a body as disclosed above and discussed in further detail below.

The objects are furthermore achieved by a body as indicated above and further discussed below for use in therapeutic applications: for use as a medicament or for use in surgery, therapy and/or in vivo diagnostics, preferably for use in the treatments of cancer.

Accordingly, a first aspect of the invention is directed to such body comprising an oxide of yttrium supported on a sulfur-containing carbon-based particle, wherein said body comprises yttrium to sulfur in an atomic ratio ranging from 1.00:0.01 to 1:10.

A process for producing a body preferably comprises the steps of:
- contacting a carbon source material, wherein preferably said carbon source material comprises at least one sulfur group, with an aqueous solution of a salt of yttrium, thereby producing a modified carbon source material;
- drying the modified carbon source material, wherein the contacting and drying may be performed several times (two or more times); and,
- subjecting said dried modified carbon source material to pyrolysis under inert conditions.

The term "body of the invention" refers to the body contained in the pharmaceutical composition and to the body for use in therapeutic applications.

The term "or" as used herein is defined as "and/or" unless specified otherwise.

The term "a" or "an" as used herein is defined as "at least one" unless specified otherwise, or it follows from the context that it should refer to the singular only.

When referring to a noun (e.g. a body, a salt, an element, etc.) in the singular, the plural is meant to be included, or it follows from the context that it should refer to the singular only.

The term "substantial(ly) or "essential(ly)" is generally used herein to indicate that it has the general character or function of that which is specified. When referring to a quantifiable feature, these terms are in particular used to indicate that it is for at least 75%, more in particular at least 90%, even more in particular at least 95% of the maximum that feature.

The term "low pH" as used herein is defined as an "acidic pH", i.e. the pH is less than 7.

The term "room temperature", as used herein, is defined as a temperature of about 25 °C.

The term "cancer", as used herein, refers to a malignancy, such as a malignant tumor, which is typically a mass of tissue that is present (e.g. in an organ (for example: brain, kidney, liver, pancreas, skin, lungs, heart, intestines, stomach, thyroid, parathyroid gland etc.), or the lymph system) of the human or animal body. The terms "cancer" and "tumor" are used interchangeably herein.

The term "individual", as used herein, is defined herein as "the human or animal body".

Preferably, the body of the invention comprises yttrium to sulfur in an atomic ratio of at least 1.0:0.1 and more preferably at least 1.0:0.4. Preferably, the body of the invention comprises yttrium to sulfur in an atomic ratio of at most 1:5 and more preferably at most 1:3.5.

The weight ratio of yttrium to sulfur is preferably in the range of from 1.0 to 5.0:1, more preferably of from 1.5 to 3.5:1, most preferably from 2.0 to 3.0:1.

The body of the invention is a composite material typically comprising yttrium and sulfur in a total amount of at least 3 wt%, preferably at least 7 wt%, and more preferably at least 10 wt%, even more preferably at least 14 wt%, in particular at least 20 wt%, and even more in particular at least 23 wt%, calculated as the total amount of elemental yttrium and elemental sulfur based on the weight of said body. As an upper limit, the body typically comprises yttrium and sulfur in a total amount of at most 90 wt%, preferably at most 75 wt%, even more preferably at most 60 wt%, in particular at most 45 wt%, more in particular at most 40 wt%, and even more in particular at most 30 wt%, calculated as the total amount of elemental yttrium and elemental sulfur based on the weight of said body which is 100 wt%.

Typically, the body of the invention comprises elemental carbon in an amount of 5 to 80 wt%, preferably 10 to 80 wt%, more preferably 20 to 70 wt% and even more preferably 40 to 70 wt%, based on the weight of said body which is 100 wt%. Preferably, the elemental carbon present in said body is in the form of amorphous carbon, graphitic carbon and combinations therefore, and preferably amorphous carbon.

The remainder of the body is typically oxygen (O), hydrogen (H) or nitrogen (N) in case dinitrogen or nitrogen containing components have been used during the preparation of the spheres. The amounts can be such that the sum of the wt% of all ingredients of the body is 100 wt%. Thus, the sum of the cited amounts adds up to 100 wt%.

The carbon based particle of the body of the invention, in addition to comprising elemental carbon, also typically comprises a carbon source material. The carbon source material is preferably functionalized with at least one sulfur (containing) group selected from the list consisting of sulfonic acid, sulfoxide, sulfate, sulfite, sulfone, sulfinic acid, thiol, thioether, thioester, thioacetal, thione, thiophene, thial, sulfide, disulfide, polysulfide and sulfoalkyl (e.g. sulfobutyl) groups, and combinations thereof, and more preferably a sulfonic acid group.

The carbon source material may be a polymeric matrix, wherein preferably said polymeric matrix is partially cross-linked, in particular cross-linked in an amount of 1 to 20%, and more in particular cross linked in an amount of 2 to 10%. In a particular embodiment, the polymeric matrix is an ion exchange resin, preferably a cation exchange resin, and more preferably a cation exchange resin comprising an aliphatic polymer, such as polystyrene. One particularly preferred cation exchange resin is a styrene/divinylbenzene copolymer resin.

The carbon source material may also be a material selected from the group consisting of cellulose, such as microcrystalline (MCC); cellulose-like material, such as cotton; carbohydrate, such as sugar or chitosan; active carbon; and, combinations thereof.

The advantage of the body of the invention is that the presence of sulfur in the carbon based particle (i.e. support/carrier) is that it enhances the stability of the yttrium oxides in the body, particularly in the presence of ionic components (e.g. salts) or in acidic conditions. Without wishing to be bound to by theory, it is believed that sulfur in the body (e.g. sulfate or sulfoxide) is at least partly responsible for a good distribution of the yttrium throughout the body. In addition, the presence of thiophenic (and sulfite) functionalities as a result of the heat treatment, will enhance the incorporation of the yttrium oxides into the body. This effectively prevents or substantially limits the leaching out (extraction) of the yttrium from the body in a liquid, such as an aqueous solution or a biological fluid (e.g. blood), in particular at low pH or in the presence of cations and anions.

Another advantage of the body of the invention is that the carbon in the carbon based carrier functions as a neutron moderator, which is relatively stable against neutron irradiation. Carbon also is typically resistant to modification of its shape (i.e. keeps its shape) and substantially chemically inert. Further, the surface of the carbon may be functionalized according to known methods in the art.

In a specific embodiment, the yttrium at least partially comprises a radioactive isotope of said yttrium. The radioactive isotope of said yttrium may be generated by numerous methods, a non-exhaustive list includes neutron irradiation, laser pulse generation, laser-plasma interaction, cyclotron and using other sources of neutrons or charged atoms. For example, upon neutron irradiation ⁸⁹Y is converted to ⁹⁰Y. The body of the invention may suitably be a radioactive body. Preferably, however, the body of the invention is initially non-radioactive (i.e. prior to use in a medical application), which has the advantage in that it avoids that personnel are exposed to radiation and avoids the need for specially equipped facilities, such as hot cells and transport facilities.

Yttrium is typically employed in the form of the oxide Y₂O₃.

Yttrium oxide can be derived from gadolinite Y₂FeBe₂O₂[SiO₄]₂, from thalinite Y₂[Si₂O₇], xeno-time [YPO₄] and euxenite.

Typical yttrium salts which may be employed to prepare the yttrium oxides can be yttrium halogenides, like yttrium fluoride YF₃ and yttrium chloride YCl₃ as well as yttrium sulfate Y₂(SO₄)₃ and yttrium nitrate Y₂(NO₃)₃. Also yttrium hydroxide Y(OH)₃ can be employed as a precursor material. The oxides can also be obtained starting from yttrium hydride YH₃.

Another starting material is yttrium oxalate.

Typically, in the body of the invention, the oxide of yttrium is in the form of particles, and preferably nanoparticles. The nanoparticles of the oxide of yttrium more preferably have a diameter of 10 nm or less, and even more preferably have a diameter which is 5 nm or less. The diameter of the nanoparticles, as used herein, is typically the value that can be determined by X-ray Diffraction unless otherwise indicated. Typically, the diameter of the nanoparticle is calculated from the peak width of the diffraction pattern of a specific component using the Scherrer equation. The diameter of the nanoparticles may also be suitably determined by transmission electron microscopy (TEM) or scanning electron microscopy (SEM). The yttrium oxide (nano)particles are typically present on the surface of the sulfur-containing carbon-based particle, which surface includes the (inner) pore area.

The body of the invention can be tailored to any desired size from several tens of nanometers up to a millimeter dependent upon its intended application. Typically, the size (i.e. diameter) of the body is at least 0.01 µm, preferably at least 0.05 µm, more preferably at least 0.1 µm, even more preferably at least 1 µm and in particular at least 15 µm. The body also typically has a diameter of at most 500 µm, preferably at most 400 µm, more preferably at most 300 µm, even more preferably at most 200 µm, in particular at most 100 µm, and more in particular at most 60 µm. Bodies of the invention can thus have a diameter of 0.01 to 500 µm, preferably 1 to 400 µm, more preferably 1 to 300 µm and even more preferably 1 to 200 µm, and in particular 15 to 60 µm, or having a diameter of 1 to 100 µm preferably 1 to 50 µm, more preferably 1 to 30 µm and even more preferably 5 to 20 µm. The size of the body, as used herein, is preferably the value as measured according to International Standard ISO 13319 on a Multisizer 3 Coulter Counter, Beckman Coulter, equipped with a 100 µm orifice for the 1 to 70 micron and for larger particles the 300 or 500 µm orifice. Smaller particles, smaller than 5 micron, can be measured with for example laser diffraction methods. Preferably, the size is the volume average diameter of the body, e.g. the d₅₀-value.

The diameter of the body of the invention typically refers to a spherical particle. In case the shape of the body deviates from spherical, the diameter refers to the largest dimension of the particle. Preferably the body of the invention is spherical or essentially spherical, in particular having a sphericity of close to 1, for instance more than 0.75, preferably more than 0.85 and more preferably at least 0.95. The sphericity of a certain particle is the ratio of the surface area of a sphere having the same volume as said particle to the surface area of said particle.

Although the yttrium oxide particles in the body of the invention are very small, the crystal structure can be the same as the normally occurring crystal structure of the bulk oxide material, which is cubic.

Typically, the body of the invention has a density which is tailored to the intended use. Typically, when used in medical applications, in particular in cancer treatment, the body of the invention has a density of > 0.8 g/ml to 8.0 g/ml, preferably about 0.9 to 6.0 g/ml, more preferably 1.0 to 4.0 g/ml, and even more preferably 1.0 to 3.5 g/ml, in particular 1.1 to 3.0 g/ml, and more in particular 1.2 to 2.5 g/ml. The density may be measured by He-picnometry or H₂O-picnometry. The advantage of such a low density for the body of the invention is that this makes it compatible with the density of biological fluids, such as a blood stream. This in turn leads to a substantially homogeneous distribution of the body within a target organ, which prevents or substantially minimizes the occurrence of focal areas of excessive concentration.

In a preferred embodiment, the body of the invention comprises one or more active and/or hydrophilic groups (chemically) attached to/are present on the surface of the body. Such hydrophilic groups may be selected from sulfonic acid groups, hydroxyl groups, carbonyl groups, carboxyl groups, sulfhydryl groups, amino groups, polyaromatic groups and combinations thereof, preferably hydroxyl, carboxyl and/or sulfonic acid groups. Such active groups may be selected from antibodies, nucleic acids, lipids, fatty acids, carbohydrates, polypeptides, amino acids, proteins, plasma, antigens, liposomes, hormones, markers and combinations thereof.

In another preferred embodiment, the body of the invention contains or is at least partly, preferably completely, coated by a layer of an element or an element oxide (i.e. an oxide of an element), wherein said element is selected from the group consisting of silicon, titanium, zirconium, hafnium, cerium, aluminium, niobium, tantalum and combinations thereof. The content of these elements in the body is preferably in the range of from 0.1 to 40 wt%, more preferably 0.5 to 30 wt%, most preferably 1 to 25 wt%, based on the weight of the body. The sum of the amounts of the other elements (Y, S, C, O, H, N) mentioned above is consequently lowered in this case to preferably 60 to 99.9 wt%, more preferably 70 to 99.5 wt%, most preferably 75 to 99 wt%, based on the weight of the body, while their weight ratio remains the same. More preferably, said layer coating of said body is of a substantially uniform thickness. The advantages of the body of the invention further comprising such a coating is that it can provide stability against leaching of components (e.g. elements, etc.) from the body, influences the hydrophilicity of the body, and/or enables tuning of the density of said body, particularly when used in combination with selective oxidation (i.e. carbon is at least partly removed) of the coated body. Selective oxidation may also be applied to the uncoated body; or, to the coated or uncoated carbon source material (optionally further comprising a salt of yttrium) prior to pyrolysis. A further advantage of such a coating is that it might provide additional biological inertness, e.g. to avoid coagulation in blood or diminish toxicological or mutagenic activity in the (human or animal) body.

In a further preferred embodiment, the body of the invention also comprises at least one element selected from the group consisting of iron, holmium, gadolinium, manganese, phosphorous, iodine, iridium, rhenium and combinations thereof. The advantage of the body further comprising iron, holmium, manganese and/or gadolinium is that these elements are paramagnetic, which enhances the therapeutic and diagnostic effectiveness of the body when used as a medicament in surgery or therapy and diagnostic methods. The advantage of using phosphorous, iodine, iridium, holmium and/or rhenium is that the radionuclides of these elements have a radioactive decay particularly suitable for medical applications.

The body of the invention also contains oxygen, e.g. in the form of Y₂O₃. The oxygen amount is adjusted so that the total amount of all elements (including carbon, yttrium, sulfur, hydrogen, nitrogen and additional elements) of the body of the invention is 100 wt%.

The process for the preparation of the body of the invention can comprise the steps of:
- contacting a carbon source material, wherein preferably said carbon source material comprises at least one sulfur group, with an aqueous solution of a salt of yttrium thereby producing a modified carbon source material;
- drying the modified carbon source material; and,
- subjecting said dried modified carbon source material to pyrolysis under inert conditions.

In a preferred embodiment, the process comprises introducing yttrium cations into the carbon source material, wherein preferably said carbon source material comprises at least one sulfur group, by ion exchange by contacting it with the aqueous solution of the salt of said yttrium, thereby producing a modified carbon source material.

In another preferred embodiment, the process comprises impregnating the carbon source material, wherein preferably said carbon source material comprises at least one sulfur group, with the salt of said yttrium by contacting it with the aqueous solution of the salt of said yttrium, thereby producing the modified (i.e. impregnated) carbon source material. The impregnation method suitable to be used may be incipient wetness, wet impregnation or vacuum impregnation. The impregnation step may also be performed on a modified carbon source material that has been obtained, e.g. after the introduction of the yttrium by ion exchange.

Optionally, the modified carbon source material is washed in a washing step with a liquid prior to the pyrolysis step. Suitable liquids may include water and/or an alcohol, such as isopropanol. The advantage of carrying out this washing step is that it removes any residual salts (i.e. non-ion exchanged yttrium salts) and other components as a result of the ion exchange process, such as HCl, NaCl, NaNO₃, HNO₃, or ammonium salts which may be present in the modified carbon source material.

In a further preferred embodiment, the process comprises depositing yttrium by precipitation onto the carbon source material, wherein preferably said carbon source material comprises at least one sulfur group, by contacting it with the aqueous solution of the salt of said yttrium, thereby producing the modified carbon source material.

A suitable carbon source material for use in the process may be a polymeric matrix, wherein preferably said polymeric matrix is partially cross-linked, in particular cross-linked in an amount of 1 to 20%, and more in particular cross linked in an amount of 2 to 10%. In a particular embodiment, the polymeric matrix is an ion exchange resin, preferably a cation exchange resin, and more preferably a cation exchange resin comprising an aliphatic polymer, such as polystyrene. One particularly preferred cation exchange resin is a styrene/divinylbenzene copolymer resin.

The carbon source material suitable for use in the process may also be selected from the group consisting of cellulose, such as microcrystalline (MCC); cellulose-like material, such as cotton; carbohydrate, such as sugar or chitosan; active carbon; and, combinations thereof.

Preferably, the carbon source material comprises at least one sulfur (containing) group, preferably on the surface of said carbon source material, and more preferably the sulfur (containing) group is selected from the group consisting of sulfonic acid, sulfoxide, sulfate, sulfite, sulfone, sulfinic acid, thiol, thioether, thioester, thioacetal, thione, thiophene, thial, sulfide, disulfide, polysulfide and sulfoalkyl (e.g. sulfobutyl) groups, and combinations thereof, even more preferably a sulfonic acid group.

The carbon source material may optionally be pretreated prior to use in the method by rinsing with water of an organic solvent, such as an alcohol or acetone. Another optional pre-treatment may be contacting the carbon source material via ion exchange with an aqueous solution comprising at least one soluble salt, thereby replacing at least part of any cations present in the carbon source material. Suitable soluble salts which could be used include ammonium chloride or sodium chloride. It is believed that such a pretreatment could influence the distribution of the yttrium oxides in the body and/or the porosity/density of the resulting body.

Suitable yttrium salts which may be used in the process are soluble yttrium salts including sulfate, nitrate, fluoride, chloride, hydroxide, hydride, phosphate and/or organic salts (e.g. acetate and citrate). Preferably, the yttrium salt is yttrium chloride or yttrium nitrate.

Preferably, the contacting step of the process is carried out at a temperature of between 15 °C and 100 °C, more preferably between 20 °C and 60 °C.

Typically, the drying step is carried out until the dried product reaches constant weight. Preferably the drying is carried out at least at room temperature, and more preferably at a temperature of between 80 and 150 °C. The drying step may be carried out in more than one step.

The pyrolysis step temperatures may be carried out at a temperature of at least 200 °C, preferably at least 300 °C, more preferably at least 600 °C, even more preferably at least 700 °C, and in particular at least 800 °C. The maximum temperature suitable to use for the pyrolysis step is typically at most 2000 °C, preferably at most 1500 °C, and more preferably at most 1000 °C. This step is carried out under inert conditions, that is under conditions that avoid reaction of carbon with the surroundings. Preferably these conditions comprise exclusion of oxygen from air. This may preferably be obtained by carrying out the pyrolysis under a typical "inert" gas, such as nitrogen or a noble gas, such as argon or helium, which is used to dissipate the oxygen containing air. During the pyrolysis of the carbon source material the size of the particles is decreased. Typically, the size of the carbon source is reduced by 5 to 50 % in diameter, more typically between 10 to 40 %. Precise control of the diameter of the particles is possible by controlling of the pyrolysis conditions (i.e. temperature, duration and gas composition).

Optionally, the pyrolyzed modified carbon source material is subjected to a post treatment. One suitable post treatment comprises the step of mixing the pyrolyzed modified carbon source material with an excess of liquid (i.e. water) comprising a surfactant to form a suspension, subjecting the suspension to agitation, filtering the suspension, washing and then drying the post treated pyrolyzed modified carbon source material. Suitable surfactants which may be used include non-ionic and anionic surfactants. The agitation step may be carried out by using a stirrer or a mixer, such as an ultrasonic mixer. The washing and drying steps are as described herein above. The advantage of this step is that it removes any water soluble byproducts formed during the pyrolysis step and helps to take apart assembled particles into individual particles without damaging the surface.

Another suitable post treatment comprises a heat treatment using a different gas composition compared to the gas composition of the pyrolysis step. For example, air or oxygen mixed with N₂, can be used at elevated temperatures to partially oxidize the pyrolyzed modified carbon source material.

In a preferred embodiment, the process further comprises loading the carbon source material with a precursor of other elements selected from the group consisting of iron, holmium, gadolinium, manganese, phosphorous, iodine, iridium, rhenium and combinations thereof. The precursor of these other elements may be loaded by methods known in the art, such as, ion exchange, impregnation and/or deposition precipitation.

Preferably, the process comprises an additional step in which the body of the invention is functionalized by attaching one or more active and/or hydrophilic groups to the surface of the sulfur-containing carbon-based particle. Since the surface of said particle typically comprises graphitic and/or amorphous carbon, attaching chemical groups to the surface is relatively easy using techniques known in the art. Such hydrophilic groups and active groups correspond to those groups as mentioned herein above.

In another preferred embodiment, the process further comprises at least partly, preferably completely, coating the carbon source material either prior to or after contacting the carbon source material with an aqueous solution of a salt of yttrium; or, the body of the invention, in particular the oxide of yttrium of the body; by a layer of an element or element oxide (i.e. an oxide of an element). Suitable elements are selected from the group consisting of silicon, titanium, zirconium, hafnium, cerium, aluminium, niobium, tantalum and combinations thereof.

The layer of the element or element oxide may be applied to the carbon source material either prior to or after contacting the carbon source material with an aqueous solution of salt of yttrium; or, to the body, in particular the oxide of yttrium of the body, by suitable means. One such suitable means includes using the sol-gel method. Suitable starting materials for use in the sol-gel method include alkoxides, chlorides or a stabilized sol of said elements (i.e. silicon, titanium, zirconium, hafnium, cerium, aluminium, niobium and/or tantalum). Typically, in the sol-gel method the pH is adjusted to either be more basic or acidic using compounds known in the art. Such suitable compounds include ammonia, aqueous ammonium, hydroxide solution, alkali hydroxides, fluoride salts or mineralic acids. More preferably, said layer coating of said body is of a substantially uniform thickness.

The process may include a following step in which carbon is at least partly removed (i.e. selective oxidation) from the coated or uncoated body; or, from the coated or uncoated carbon source material (optionally further comprising a salt of yttrium) prior to the pyrolysis step. Typically, the carbon may be removed in a calcination step, wherein the coated or uncoated body of the invention; or, the coated or uncoated carbon source material (optionally further comprising a salt of yttrium) prior to the pyrolysis step; is calcined in an oxygen containing gas flow at a temperature of 900 °C or less, preferably 600 °C or less, more preferably 500 °C or less, and even more preferably 400 to 500 °C.

The body of the invention may be used as a medicament (such as a pharmaceutical). In particular, the body of the invention is used in the preparation of a pharmaceutical or a pharmaceutical composition (preferably for the treatment of a medical disorder (i.e. disease/condition, such as cancer)).

The pharmaceutical composition comprises a body of the invention as described above.

The pharmaceutical composition can furthermore contain pharmaceutically acceptable adjuvants or further pharmaceutically acceptable ingredients, e.g. for tailoring the pharmaceutical compositions. Further ingredients may for example be suspending agents, pH-adjusting agents, diluents, etc. Further adjuvants or further ingredients are listed below.

Preferably, the pharmaceutical composition comprises a suspension or dispersion of the bodies according to the present invention in a suitable (liquid) suspension or dispersion medium (carrier) which is biocompatible and especially compatible with the human body and with blood.

When in the following reference is made to the body according to the present invention, this term shall also encompass the pharmaceutical composition according to the present invention. Thus, when it is described that the body is used for a specific application, it is understood that as well the pharmaceutical composition comprising the body can be used for the application.

Thus, the pharmaceutical composition comprises a body as disclosed above. The pharmaceutical composition preferably comprises 0.1 to 99 wt%, more preferably 2 to 50 wt%, of at least one body of the invention, based on the total weight of the pharmaceutical composition.

The remainder can be pharmaceutically acceptable carriers, adjuvants, diluents, excipients or other additional components.

The pharmaceutical composition can be a pharmaceutical composition for the treatment of a disease/condition, preferably for the treatment of cancer.

The invention also relates to a body as discussed above for use in the treatment of a disease/condition, preferably for the use in the treatment of cancer or infectious tissue (for example an implantation that is contaminated by microbes), or an epileptic center in the brain.

In the treatment of cancer, the body according to the invention or the pharmaceutical composition according to the invention is administered to the human or animal body.

Preferably, said body is used (preferably as a medicament) in a method for the treatment of the human or animal body.

More preferably, said treatment is a method of surgery, therapy and/or in vivo diagnostics. More in particular, the method of surgery, therapy and/or in vivo diagnostics comprises:
- imaging, such as magnetic resonance imaging, nuclear scanning imaging, X-ray imaging, positron emission tomography (PET) imaging, single-photon emission computed tomography (SPECT) imaging, X-ray computed tomography (CT) imaging, scintigraphy imaging, ultrasound, and/or fluorescent imaging;
- radiotherapy, preferably radio-embolization.

Preferably, the surgery, therapy and/or in vivo diagnostics is a method for detection and/or treatment of a cancer, in particular a cancer of the brain, pancreas, lymph, lung, head and neck, prostate, breast, liver, intestines, thyroid, stomach, or kidney.

Preferred is a use of the body, wherein the body is administered as a medical device or medicament by catheter, injection, preferably intravenous or direct injection, infusion, or, a skin patch, or, wherein the body is administered in a method of surgery, therapy and/or in vivo diagnostics by catheter, injection, preferably intravenous or direct injection, infusion, or, a skin patch.

Said body can be detected by methods well known in the art such as positron emission tomography (PET), single photon emission computed tomography (SPECT) and gamma camera's to image Brehmstrahlung.

Preferably, said body or pharmaceutical composition is in the form of a suspension. The meaning of the word suspension, as used herein, should also be understood as at least including dispersions. Typically, the suspension comprises the body of the invention and a (carrier) fluid or gel. Suitable (carrier) fluids which may be used in said suspension include aqueous solutions, such as a saline solution (i.e. sodium chloride in water), a phosphate buffered saline (PBS) solution, sugar solution, saccharose containing solutions (e.g. dextrose in water) or a tris buffered saline solution. Optionally, said aqueous solution also comprises additional ion or non-ionic, surface active ingredients, i.e. surfactants. A suitable gel for use in said suspension is a dextran, alginate or gelatin or starch derived gel.

The body or pharmaceutical composition used according to the invention may be administered as a medicament; or, in a method of surgery, therapy and/or in vivo diagnostics by suitable means, such as by catheter (for example radioembolisation of liver tumors), (direct or intravenous) injection, infusion, a patch on the skin of an individual (i.e. a skin patch), etc.

Magnetic resonance imaging (MRI) provides information of the internal status of an individual. A contrast agent is often used in order to be capable of obtaining a scanning image. For instance iron, holmium and gadolinium, preferably in the form of ferrite particles, holmium oxide particles and gadolinium-DTPA (diethylaminetriaminepentaacetic acid) complexes, are often used in contrast agents for MRI scanning. This way, a good impression can be obtained of internal disorders, like the presence of (a) tumor(s). After diagnosis, a treatment is often started involving administration of a pharmaceutical composition to an individual. It is often important to monitor the status of the individual during treatment as well. For instance, the course of a treatment and targeting of a drug can be monitored, as well as possible side effects which may imply a need for terminating, or temporarily interrupting, a certain treatment.

The body of the invention may be used in a method for detecting cancer, which method comprises the steps of:
- administering the body of the invention to an individual;
- obtaining a scanning image; and
- determining whether said image reveals the presence of a cancer.

The body of the invention may suitably be administered in said method of detecting cancer in the form a suspension, as described herein above.

Sometimes local treatment in only a specific part of the individual is preferred. For instance, tumor growth can sometimes be counteracted by a method of internal radiotherapy comprising administration of a radioactive body to an individual. If said radioactive body accumulates inside and/or around the tumor, specific local treatment is possible.

In a preferred embodiment, the body or pharmaceutical composition of the invention, when administered intravenously via an injection in the blood vessel and which accumulates in the cancer due to the enhanced permeability and retention (EPR) effect, typically has a diameter in the range of less than 1 µm, preferably from 0.01 to 0.50 µm, and more preferably 0.05 to 0.30 µm.

The body of the invention, when administered to a cancer, e.g. liver cancer, via a catheter, typically has a diameter in the range of 1 to 400 µm, preferably 1 to 200 µm, more preferably 1 to 100 µm and even more preferably 10 to 60 µm.

The body of the invention, when administered to a cancer via a direct injection (i.e. intratumoral injection), typically has a diameter in the range of 1 to 100 µm, preferably 1 to 50 µm, more preferably 1 to 30 µm and even more preferably 5 to 20 µm. Such bodies can attractively be used for local therapeutic and in addition diagnostic purposes. For local therapeutic purposes the body(ies) can suitably be delivered (i.e. administered) locally via a catheter or via direct injection, whereas for diagnostic purposes the body(ies) can be introduced (i.e. administered) into a human or animal body via parenteral administration, e.g. via injection, infusion, etc.

The diameter is determined as described above.

The body or pharmaceutical composition of the invention may also be used in a method for treating cancer, which method comprises the steps of:
- administering the body of the invention to an individual;
- obtaining a scanning image of the individual; and
- determining the distribution of said body of the invention within the individual; and,
- administering to the individual a therapeutic composition comprising said body of the invention.

The body of the invention in said therapeutic composition is typically provided with at least one active group. Preferably, the body of the invention and the therapeutic composition suitable for use in said method of treating cancer are each in the form of a suspension, as described herein above.

The pharmaceutical composition can comprise a body of the invention as the sole pharmaceutically active ingredient. It is also possible that the pharmaceutical composition additionally contains suspended bodies comprising pharmaceutically active elements different from yttrium, e.g. holmium (in oxide form) on a similar or identical support. In the pharmaceutical composition, up to 99 wt% of the yttrium of the invention can be replaced by these other isotopes.

Thus, one can use a physical mixture (cocktail) of spheres containing different elements. One could e.g. apply a mixture of spheres in which 50 % contain holmium and the other 50 % contains yttrium. One advantage is that a wanted grade of radiation can be obtained (depending on nuclear cross section of the elements (Ho = 64, Y = 1.3 barn)). After for example 3 days of neutron bombardment, Ho will stay in its equilibrium state whereas ⁹⁰Y will still increase. Similar physical mixtures can also be obtained by using other elements that can be neutron activated, like dysprosium or rhenium.

Preferably, the form of radiotherapy used is radio-embolization, when the body is combined with radioactive bodies. Radio-embolization is a treatment which combines radiotherapy with embolization. Typically, the treatment comprises administering (i.e. delivering) the body of the invention, for instance via catheterization, into the arterial blood supply of an organ to be treated, whereby said body becomes entrapped in the small blood vessels of the target organ and irradiate the organ. In an alternate form of administration, the body of the invention may be injected directly into a target organ or a solid tumor to be treated (i.e. intratumoral injection). The person skilled in the art, however, will appreciate that the administration of the body of the invention may be by any suitable means and preferably by delivery to the relevant artery. The body may be administered by single or multiple doses, until the desired level of radiation is reached. Preferably, the body of the invention is administered as a suspension, as described herein above.

In a preferred embodiment, the method of surgery, therapy and/or in vivo diagnostics is a method of detecting and/or treating a cancer, but particularly in the treatment of brain, pancreas, lymph, lung, head and neck, prostate, intestines, thyroid, stomach, breast, liver and kidney cancers, and more in particular metastases, by administering said body. Said body may suitably be administered to cancers of the brain, pancreas, intestines, thyroid, stomach, head and neck, lung and breast cancers via an (intratumoral) injection. Said body may also be suitably administered to cancers of the liver, kidney, pancreas, brain, lung and breast via a catheter.

The body of the invention, when used in said method of detecting and/or treating of a cancer, typically tends to accumulate in cancer tissue substantially more than it does in normal tissues due to the enhanced permeability and retention (EPR) effect, particularly when the body has a size of 0.01 to 2 µm and more in particular 0.01 to 0.9 µm. It is believed that this phenomenon is a consequence of the rapid growth of cancer cells, which stimulates the production of blood vessels. Typically, vascular endothelial growth factor (VEGF) and other growth factors play a role in cancer angiogenesis. Tumor cell aggregates (i.e. tissues) having a size of 1 to 2 mm usually start to become dependent on blood supply carried out by neovasculature for their nutritional and oxygen supply. These newly formed tumor vessels are usually abnormal in form and architecture. The tumor cells are poorly aligned defective endothelial cells with wide fenestrations, lacking a smooth muscle layer, or innervation with a wider lumen, and impaired functional receptors for angiotensin II. Furthermore, the tumor tissues usually lack effective lymphatic drainage. All of these factors lead to abnormal molecular and fluid transport dynamics. The EPR effect is further enhanced by many pathophysiological factors involved in enhancement of the extravasation of the body of the invention in solid tumor tissues. One factor that lends to the increased retention is the lack of lymphatics around the tumor region which would filter out such particles (i.e. the body of the invention) under normal conditions. The EPR effect helps to carry the body of the invention and spread it inside the cancer tissue (i.e. solid tumors) enabling it to be more effective in methods of detecting and/or treating cancers.

For the purpose of clarity and a concise description features are described herein as part of the same or separate embodiments, however, it will be appreciated that the scope of the invention may include embodiments having combinations of all or some of the features described.

The various aspects of the invention are now illustrated on the basis of the following non-limiting examples.

### Examples

### Example 1 (pyrolyzed yttrium containing resin spheres)

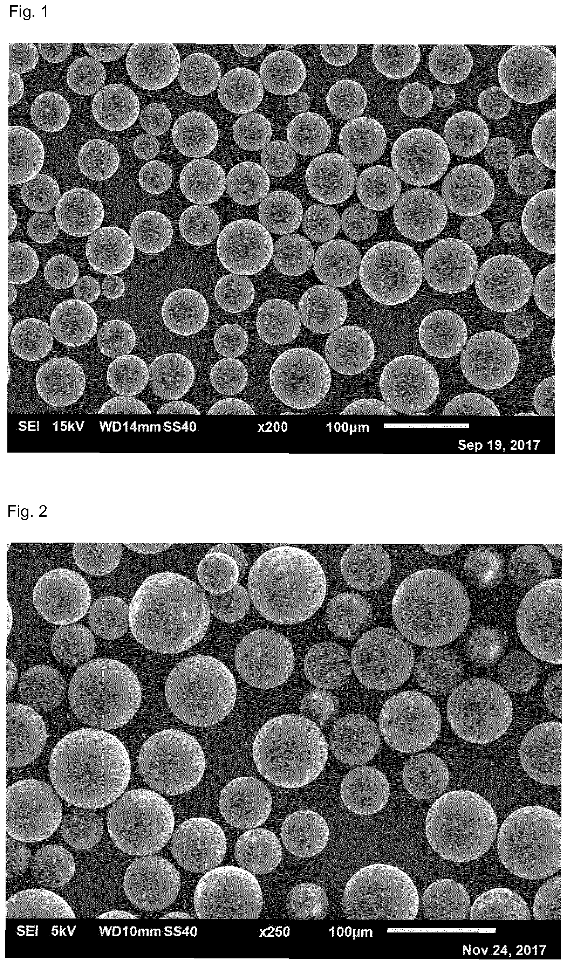

31.83 grams of Dowex® 50WX4 H+ (i.e. a styrene-divinylbenzene polymer matrix functionalized with a sulfonic acid group, Fluka, 200-400 mesh, spherically shaped particles, having a diameter between 34 and 74 micron) were washed with demi-water until the wash water was colorless. To a 200 mL slurry of the washed Dowex resin spheres in water, was added a 30 mL water solution containing 3.74 g of yttrium chloride hexahydrate (YCl₃·6H₂O, Sigma-Aldrich, 99,9% purity). The mixture was stirred for 3 hours at room temperature, which resulted in the yttrium cations being introduced into the ion exchange resin by ion exchange. Next, the beige particles were filtered and washed with 300 ml H₂O and 100 ml isopropanol (technical grade, VWR). The washed material was dried overnight in an oven at 110 °C which yielded 11.90 grams of an ochre yellow colored powder. This dried material was then pyrolyzed by heating to 800 °C (ramp 2 °C/min., hold 1 hour) under a nitrogen flow of 16.0 nl/h with fluidization (i.e. the volume of the bed is increased with a factor 2 to 3 without blowing particles out of the reactor), which produced a black powder (nl means normal liters). After the pyrolysis step was completed, the pyrolyzed material was allowed to cool to room temperature while being kept under a N₂ flow. Finally, the pyrolyzed material was treated with a flow of air 6 nl/h in a nitrogen flow of 10 nl/h with control of the exotherm (max 70 °C). When cooled down to room temperature, the reactor was emptied and the material was washed with demi-water with a few droplets of surfactant (dreft soap for dish washing, which comprises a mixture of 5 to 15 % of anionic surfactants and < 5 % nonionic surfactants). The suspension was treated in an ultrasonic bath for 60 minutes and subsequently filtered, washed with 500 mL of demi-water and finally with 100 mL i-propanol. The pyrolyzed material was dried in an oven at 110 °C for 6 hours and finally the pyrolyzed material was sieved over a 100 micron sieve to remove the larger particles. Yield: 7.1 grams of black powder. Figure 1 is a scanning electron microcopy (SEM) image of the material and Table 1 contains the elemental composition found by SEM-EDS (Scanning Electron Microscopy with Energy Dispersive Spectroscopy). SEM showed nice spherical particles with diameters between approximately 10 and 80 microns. ICP (inductively couples plasma) analysis; Y: 14.0 wt%, C = 59 wt%, S = 11 wt%, N = 0.1 wt%, density = 1.9 g/ml by H₂O picnometry. Powder X-ray diffraction (XRD) showed completely amorphous material.

**Table 1. Elemental composition found by SEM-EDS and calculated molar ratio of Example 1.**

| element | wt% | molar ratio |
|---|---|---|
| C | 63.5 | 46.6. |
| O | 22.2 | 12.2 |
| S | 4.2 | 1.2 |
| Y | 10.1 | 1.0 |

### Example 2 (pyrolized SiO₂ coated yttrium containing resin spheres)

### SiO₂ coating of resin spheres

This was done according to the following general description: 20.0 grams of cationic ion exchange resin (Dowex® 50WX4 H+, Fluka, 200-400 mesh) was washed with demi-water until the wash water was colorless. After filtration, the wet filter cake was dispersed in a 2liter round bottom flask in a mixture of ethanol and water (192 ml deionized water, 746 grams ethanol). To this mixture, 0.154 g cetyltrimethylammoniumbromide (Sigma-Aldrich 95% purity) was added and the mixture was stirred for 1 hour at room temperature and standard ambient pressure (i.e. 100 kPa). Then, 9.19 mL of a 25% ammonia solution was added and the mixture was stirred for an additional 15 minutes. To this was drop wisely added (in 1 hour) an ethanolic tetraethyl orthosilicate solution (12.58 ml in 150 ml ethanol). After stirring for 18 h at room temperature and standard ambient pressure (i.e. 100 kPa) the material was separated, washed two times with water and ethanol (50 ml each). Finally, the material was dried in a drying oven at 60 °C for 24 hours yielding 7.42 gram an off-white powder. Thermographic analysis showed that the sample contained 2.1 wt% of SiO₂.

### Yttrium loading

3.00 grams of SiO₂ coated ion-exchange resin spheres was loaded with yttrium by adding 4.53 grams of YCl₃.6H₂O to a 100 mL aqueous slurry of this material and stirring the slurry overnight. Next, the particles were filtered and washed with 400 mL demi-H₂O and 150 mL isopropanol (technical grade, VWR), respectively. Drying was performed in an oven at 105 °C overnight. This yielded 3.60 grams of a slightly yellow powder. This material was heated in a pyrolysis step in a fluidized bed at 800 °C for 1 hour. After cooling down, the material was treated in an air flow at 300 °C for 2 hours. This yielded 2.05 grams of a black powder that contained 17.1 wt% of yttrium as determined by ICP analyses. Figure 2 is a scanning electron microcopy (SEM) image of the material and Table 2 contains the elemental composition found by SEM-EDS. SEM showed nice spherical particles with diameters between approximately 10 and 80 microns. XRD showed the material to be completely amorphous. ICP analyses determined Y = 16.6 wt%. CNS analyses results: C = 49.7, N = 0.2, S = 11.2 wt%. H2O picnometry measurement showed a density of 2.1 g/ml.

**Table 2. Elemental composition found by SEM-EDS and calculated molar ratio of Example 2.**

| element | wt% | molar ratio |
|---|---|---|
| C | 44.7 | 19.4 |
| O | 29.4 | 9.6 |
| S | 6.5 | 1.1 |
| Si | 2.4 | 0.4 |
| Y | 17.1 | 1.0 |

### Analysis

ICP analyses were performed using a Thermo-Scientific iCAP 7000 series. Sample preparation was carried out by dissolving the particles in a concentrated HNO₃ solution (Lps, 65% Pro Analysis (P.A.) in a microwave at 230 °C. The yttrium content of the solutions (10 wt% HNO₃ matrix) was then measured at 324.228 and 377.433 nm after calibration. SEM-EDS analyses were carried out with a JEOL JSM-6010LA Analytical scanning electron microscope using a specifically designed EDS detector.

Carbon, nitrogen and sulfur (C, N, S) analyses were performed on a EuroVector Euro EA elemental analyzer with additional vanadium pentoxide added to the samples to assure complete oxidation of the samples.

Powder X-ray diffraction (XRD) was performed using a Bruker D8 ADVANCE (Detector: SOL'X, Anode: Copper, wavelength: 1.542 Å, Primary Soller slit: 4°, Secundary Soller slit: 4°, Detector slit: 0.2 mm, Spinner: 15 RPM, Divergence slit: variable V20, Antiscatter slit: variable V20, Start: 10° 2 theta, Stop: 100° 2 theta, Stepsize: 0.05° 2 theta, Time/step: 8 sec, Sample preparation: Front loading).

### Example 3 (pyrolyzed yttrium containing resin spheres with high S:Y ratio)

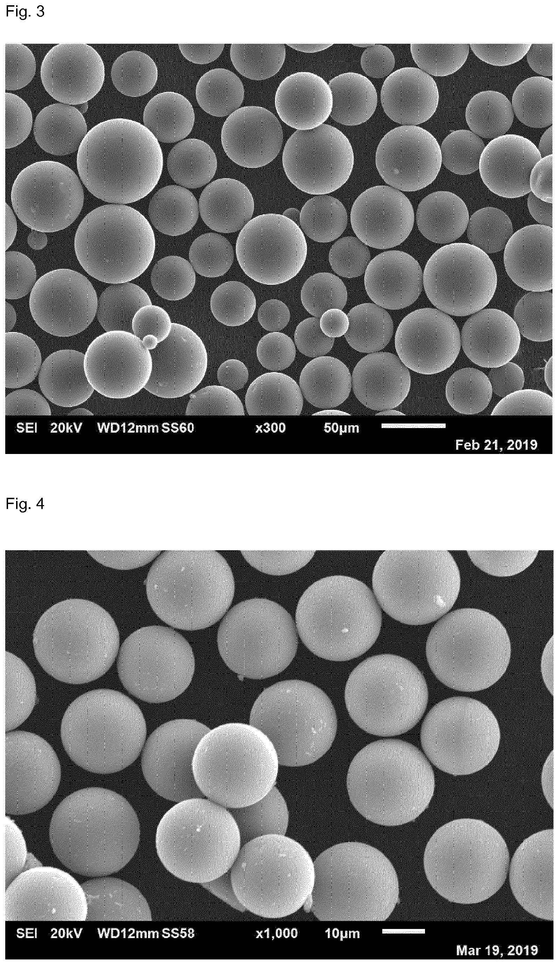

31.70 grams of Dowex® 50WX4 H+ (i.e. a styrene-divinylbenzene polymer matrix functionalized with a sulfonic acid group, Fluka, 200-400 mesh, spherically shaped particles, having a diameter between 34 and 74 micron) were washed with demi-water until the wash water was colorless. To a 230 mL slurry of the washed Dowex resin spheres in water, was added a 20 mL water solution containing 0.49 g of yttrium chloride hexahydrate (YCl₃·6H₂O, Sigma-Aldrich, 99,9% purity). The mixture was stirred for 0.5 hours at room temperature, which resulted in the yttrium cations being introduced into the ion exchange resin by ion exchange. Next, the beige particles were filtered and washed with 300 ml H₂O and 100 ml isopropanol (technical grade, VWR). The washed material was dried overnight in an oven at 110 °C which yielded 11.90 grams of an ochre yellow colored powder. This dried material was then pyrolyzed by heating to 800 °C (ramp 2 °C/min., hold 1 hour) under a nitrogen flow of 31.3 nl/h with fluidization (i.e. the volume of the bed is increased with a factor 2 to 3 without blowing particles out of the reactor), which produced a black powder (nl means normal liters). After the pyrolysis step was completed, the pyrolyzed material cooled to room temperature while being kept under a N₂ flow. Finally, the pyrolyzed material was treated with a flow of air 6 nl/h in a nitrogen flow of 10 nl/h with control of the exotherm (max 45 °C). When cooled down to room temperature, the reactor was emptied and the material was washed with demi-water with a few droplets of surfactant (Dreft soap for dish washing, which comprises a mixture of 5 to 15 % of anionic surfactants and < 5 % nonionic surfactants). The suspension was treated in an ultrasonic bath for 60 minutes and subsequently filtered, washed with 500 mL of demi-water and finally with 100 mL i-propanol. The pyrolyzed material was dried in an oven at 110 °C for 6 hours and finally the pyrolyzed material was sieved over a 100 micron sieve to remove the larger particles. Yield: 7.1 grams of black powder. Figure 3 is a scanning electron microcopy (SEM) image of the material and Table 3 contains the elemental composition found by SEM-EDS (Scanning Electron Microscopy with Energy Dispersive Spectroscopy). SEM showed nice spherical particles with diameters between approximately 10 and 70 microns. ICP (inductively coupled plasma) analysis; Y: 3.4 wt%. CNS analyses; C = 86.3 wt%, N = 0.7 wt%, S = 3.2 wt%.

**Table 3. Elemental composition found by SEM-EDS of Example 3.**

| element | wt% | Molar ratio |
|---|---|---|
| C | 89.9 | 298.9 |
| O | 5.9 | 14.7 |
| S | 2.0 | 2.5 |
| Y | 2.2 | 1.0 |

Figure 3 shows an SEM image of yttrium containing spherical material of Example 3.

### Example 4 (monosized yttrium loaded carbonized spheres with low S:Y ratio)

27.15 g of a suspension of Source30 (GE Healthcare Life Sciences) was placed in a beaker glass and dried in an oven at 110 °C overnight which resulted in 4.42 grams of material. To this was added dropwise a solution of yttrium nitrate (5.76 grams of Y(NO₃)₃·6H₂O in 7.1 g demi-H₂O). The wet material was placed in an oven and was dried at 110 °C overnight which resulted in a slightly brownish powder (8.06 grams). This powder was pyrolyzed at 800 °C in a nitrogen flow for 1 hour (ramp 2 °C/min, N2 flow 16 nL/h). After cooling and air stabilisation, and subsequent washing with water and isopropanol, 2.79 grams of a black powder was isolated and dried in an oven at 110 °C overnight. After sieving over a 71 micron sieve, 2.01 grams of a black powder was collected that contained 42.5 wt.% of yttrium by ICP analyses. CNS analyses; C = 34.1 wt%, N = 0.8 wt%, S = 1.0 wt%. Figure 4 shows a SEM image of the material and Table 4 contains the elemental composition found by SEM-EDS (Scanning Electron Microscopy with Energy Dispersive Spectroscopy).

**Table 4. Elemental composition found by SEM-EDS of Example 4.**

| element | wt% | Molar ratio |
|---|---|---|
| C | 50.1 | 11.1 |
| O | 14.6 | 2.4 |
| S | 0.7 | 0.06 |
| Y | 33.5 | 1.0 |
| Na | 0.8 | 0.1 |

Figure 4 shows an SEM image of yttrium containing spherical material of Example 4.

### Example 5. Leaching Test under neutral and acidic conditions

0.1 gram of the material according to Example 1, 3 and 4 were each separately immersed in a 10 mL demi-water (pH neutral i.e. pH = 7), 0.9 wt% saline solution and 3.1 wt% HNO3 solution (pH = 0.55 at room temperature). After 1 h stirring at room temperature at 400 rpm, the material was standing overnight at room temperature. After 24 hours, the material was filtered using a glass filtering apparatus with Whatman 589/1 filter paper. The yttrium content of the filtrate was then analyzed by ICP according to the method as described herein above. The results of the leaching experiments for Example 1, 3 and 4 with the measured amount of yttrium content in the filtrate in ppm by weight are the non-bracketed values shown in Table 5 below. The values shown in brackets in Table 5 below, are the yttrium content of the filtrates in weight percent, based upon the total amount of yttrium present in the 0.1 gram material of Examples 1, 3 and 4, respectively.

**Table 5**

| | Y content in ppm (wt.%) | | |
|---|---|---|---|
| Solution | Example 1 | Example 3 | Example 4 |
| Demi-water pH =7 | < 1 (< 0.07) | < 1 (< 0.3) | < 1 (< 0.02) |
| 0.9% saline | < 1 (< 0.07) | < 1 (< 0.3) | < 1 (< 0.02) |
| 3.1 wt.% HNO₃ | 94 (6.7) | < 10 (< 3.0) | 3600 (84.7) |

The examples clearly show:
(i) that bodies according to the present invention can be prepared with various yttrium to sulfur ratios.
(ii) the bodies are very stable in aqueous and ionic (saline) environment. Example 1 and 3 even show reasonable stability under strong acidic/oxidizing conditions. These results show a clear stability advantage over the prior art indicating that 0.01% to 0.4% of ⁹⁰Y is released from the microsphere after 20 minutes in water (Westcott M.A. et al. Adv. Radiation Onco. 2016, 1, 351-364).

## Claims

1. A pharmaceutical composition, comprising a body comprising an oxide of yttrium, wherein preferably the oxide of yttrium is in the form of (nano)particles, supported on a sulfur-containing carbon-based particle, wherein said body comprises yttrium to sulfur in an atomic ratio ranging from 1:0.01 to 1:10, and preferably a pharmaceutically acceptable carrier, adjuvant, diluent and/or excipient.

2. The pharmaceutical composition according to claim 1, wherein said body comprises yttrium and sulfur in a total amount of at least 3 wt%, preferably at least 7 wt%, and more preferably at least 10 wt%, even more preferably at least 14 wt%, in particular at least 20 wt%, and even more in particular at least 23 wt%, calculated as the total amount of elemental yttrium and elemental sulfur based on the weight of said body; and,
wherein said body comprises yttrium and sulfur in a total amount of at most 90 wt%, preferably at most 75 wt%, even more preferably at most 60 wt%, in particular at most 45 wt%, more in particular at most 40 wt%, and even more in particular 30 wt%, calculated as the total amount of elemental yttrium and elemental sulfur based on the weight of said body.

3. The pharmaceutical composition according to any of the previous claims, wherein the body comprises elemental carbon in an amount of 5 to 80 wt%, preferably 10 to 80 wt%, more preferably 20 to 70 wt% and even more preferably 40 to 70 wt%, based on the weight of said body;
wherein preferably the elemental carbon is in the form of amorphous carbon, graphitic carbon and combinations thereof, and wherein preferably said carbon-based particle of the body further comprises a carbon source material selected from the group consisting of a polymeric matrix; cellulose; cellulose-like material; carbohydrate; active carbon; and, combinations thereof, and preferably said carbon source material further comprises at least one sulfur group.

4. The pharmaceutical composition according to any of the previous claims, wherein in the body said yttrium at least partially comprises a radioactive isotope of said yttrium, and/or wherein the pharmaceutical composition additionally comprises bodies comprising pharmaceutically active elements different from yttrium, and/or wherein the pharmaceutical composition is in the form of a suspension of the body comprising an oxide of yttrium in a liquid suspension medium.

5. The pharmaceutical composition according to any of the previous claims, wherein the body has a diameter of 0.01 to 500 µm, preferably 1 to 400 µm, more preferably 1 to 300 µm and even more preferably 1 to 200 µm, and in particular 15 to 60 µm, or having a diameter of 1 to 100 µm preferably 1 to 50 µm, more preferably 1 to 30 µm and even more preferably 5 to 20 µm, and/or wherein said body has a sphericity of more than 0.75, preferably more than 0.85 and more preferably at least 0.95, and/or wherein said body has a density of > 0.8 g/ml to 8.0 g/ml, preferably 0.9 to 6.0 g/ml, more preferably 1.0 to 4.0 g/ml, and even more preferably 1.0 to 3.5 g/ml, in particular 1.1 to 3.00 g/ml, and more in particular 1.2 to 2.5 g/ml.

6. The pharmaceutical composition according to any of the previous claims, wherein said body further comprises on its surface one or more functional groups, in particular hydrophilic groups and/or active groups, and/or wherein the body contains or is at least partly coated by a layer of an element or an oxide of an element, wherein said element is selected from the group consisting of silicon, titanium, zirconium, hafnium, cerium, aluminium, niobium, tantalum and combinations thereof, and/or wherein said body further comprises other elements selected from the group consisting of iron, holmium, gadolinium, manganese, phosphorous, iodine, iridium, rhenium, and combinations thereof.

7. The pharmaceutical composition according to any of the previous claims, wherein the body is produced by a process comprising the steps of:
- contacting a carbon source material, wherein preferably said carbon source material comprises at least one sulfur group, with an aqueous solution of a salt of yttrium, thereby producing a modified carbon source material;
- drying the modified carbon source material, wherein the contacting and drying may be performed several times; and,
- subjecting said dried modified carbon source material to pyrolysis under inert conditions,
wherein said contacting is preferably by ion exchange, impregnation, and/or deposition precipitation, and wherein said process for producing the body can be followed by a step in which carbon is at least partly removed from the coated or uncoated body; or, from the coated or uncoated carbon source material prior to the pyrolysis step, wherein said carbon source material optionally further comprises a salt of yttrium.

8. The pharmaceutical composition according to claim 7, wherein said carbon source material is selected from the group consisting of a polymeric matrix; cellulose; cellulose-like material; carbohydrate; active carbon; and, combinations thereof, and/or wherein the carbon source material comprises at least one sulfur group, preferably on the surface of said carbon source material selected from the group consisting of sulfonic acid, sulfoxide, sulfate, sulfite, sulfone, sulfinic acid, thiol, thioether, thioester, thioacetal, thione, thiophene, thial, sulfide, disulfide, polysulfide and sulfoalkyl, preferably sulfobutyl groups, and combinations thereof, and preferably a sulfonic acid group.

9. The pharmaceutical composition according to one of claims 11 or 12, wherein the process for producing the body comprises loading the carbon source material with a precursor of other elements selected from the group consisting of iron, holmium, gadolinium, manganese, phosphorous, iodine, iridium, rhenium and combinations thereof, and/or wherein the process further comprises functionalizing the body, wherein the body is functionalized with a hydrophilic group and/or an active group, and/or wherein the process for producing the body further comprises at least partly coating the carbon source material either prior to or after contacting the carbon source material with an aqueous solution of a salt of yttrium; or at least partly coating the body, in particular the oxide of yttrium of the body; by a layer of an element or an oxide of an element, wherein said element is selected from the group consisting of silicon, titanium, zirconium, hafnium, cerium, aluminium, niobium, tantalum and combinations thereof.

10. Body as defined in any one of claims 1 to 9 for use as a medicament and/or a diagnostic agent, wherein preferably said use is in surgery, therapy and/or in vivo diagnostics, preferably in the treatment of cancer, and wherein preferably the surgery, therapy and/or in vivo diagnostics comprises:
- imaging, wherein preferably said imaging is selected from the list consisting of magnetic resonance imaging, nuclear scanning imaging, X-ray imaging, positron emission tomography (PET) imaging, single-photon emission computed tomography (SPECT) imaging, X-ray computed tomography (CT) imaging, scintigraphy imaging, ultrasound, fluorescent imaging and combinations thereof;
- drug delivery;
- cellular labeling; and/or,
- radiotherapy, preferably radio-embolization,
and/or wherein the surgery, therapy and/or in vivo diagnostics are methods for detection and/or treatment of a cancer, in particular a cancer of the brain, pancreas, lymph, lung, head and neck, prostate, breast, liver, intestines, thyroid, stomach, or kidney.

11. Body for use according to claim 10,
wherein the body is administered as a medicament by catheter, injection, preferably intravenous or direct injection, infusion, or, a skin patch, or,
wherein the body is administered in a method of surgery, therapy and/or in vivo diagnostics by catheter, injection, preferably intravenous or direct injection, infusion, or, a skin patch.

12. Suspension comprising a number of bodies as defined in any one of claims 1 to 9, said suspension being a therapeutic suspension, an MRI scanning suspension, and/or a nuclear scanning suspension.

13. Use of a body as defined in any one of claims 1 to 9 as contrast agent, such as blood pool contrast agent, for computer tomogram (CT) including dual energy CT, and magnetic resonance imaging (MRI).

14. A method for detecting cancer and/or monitoring a cancer, which method comprises the steps of:
- administering a body as defined in any one of claims 1 to 9 to an individual;
- obtaining a scanning image; and
- determining whether said image reveals the presence of cancer,
wherein preferably the body is in the form of a suspension.

15. A method for treating cancer, which method comprises the steps of:
- administering a body as defined in any one of claims 1 to 17 to an individual;
- administering to the individual a therapeutic composition comprising said body, and optionally further comprising
- obtaining a scanning image of the individual; and
- determining the distribution of said body within the individual,
preferably wherein the body in said therapeutic composition is radioactive and/or further comprises at least one active group, and/or wherein preferably the body and the therapeutic composition are administered to the individual in the form of suspensions.
